# EUROPEAN PATENT APPLICATION

(11) **EP 1 588 734 A1**
(43) Date of publication of application: **26.10.2005**
(21) Application number: 05102958.5
(22) Date of filing: 14.04.2005
(51) Int. Cl.: A61M 35/00, B65D 1/02, A61J 9/00

(54) **A single-dose receptacle for containing pharmaceutical and cosmetic products and the like**

(30) Priority: 19.04.2004 IT TO20040238
(71) Applicant: Bisio Progetti S.p.A, 15100 Alessandria (IT)
(72) Inventor: Rossi, Bruno, 15100 Alessandria (IT); Bisio, Stefano, 15100 Alessandria (IT)
(74) Representative: Gerbino, Angelo

(57) **Abstract**

The single-dose receptacle (10) for containing pharmaceutical and cosmetic products, and the like, comprises a hollow body (12) having a longitudinal axis (14) and having, at one end, a dispensing mouth (16). The receptacle (10) further comprises means which can allow the mouth (16) to be inclined with respect to the longitudinal axis (14).

## Description

The present invention relates to a single-dose receptacle for containing pharmaceutical and cosmetic products and the like.

In greater detail, the receptacle comprises a hollow body having a longitudinal axis and having, at one end, a mouth for dispensing the product contained therein.

The single-dose receptacle ensures the absence of bacterial growth in the product, because it completes its function in a single administration or, at the maximum, in two administration operations carried out in the space of a single day. This makes it possible to do without the addition of preservatives to the active ingredient of the product, even when the product is extremely liable to bacterial attack. The absence of preservatives constitutes a substantial advantage because, on the one hand, though they greatly reduce bacterial growth in the product once the receptacle has been opened, on the other hand, they are toxic not only for the bacterial cells but also for the cells of the human body, to the surface of which the product is applied.

Single-dose receptacles are typically used as containers for medicaments having local action (eye lotions and substances for dermatological treatments in general), whose action in chronic conditions must be extended over a period of time, heightening the risk of allergies or pathological changes in the surface being cared for, such as, for example, the conjunctival sac or other zones of the body, whether being skin or mucous membranes. Another typical field of use is then constituted by the containment of cosmetic products which are normally used for long-term treatments, so that the risk of the occurrence of problems owing to contact with preservatives is even more accentuated than in the case of pharmaceutical products.

The object of the present invention is to provide a single-dose receptacle which allows administration of the product contained therein at well-defined locations of the human body, even if they are not readily accessible, and the application thereof with greater efficiency.

According to the invention, this object is achieved by means of a receptacle of the type mentioned in the introduction to the present description and characterized in that it comprises means which can allow the above-mentioned mouth to be inclined with respect to the longitudinal axis of the receptacle body.

This possibility to incline the dispensing mouth allows application to be carried out at the locations effectively concerned, even if they are accessible only with difficulty, preventing a more extensive surface-area from being affected with the possible occurrence of undesirable side-effects.

Specific examples of products for which the precision of application is critical and for which it is therefore particularly advantageous to use the receptacle of the invention are constituted by products for dental care or oral hygiene in general, subcutaneous adhesives and healing products in general.

The subject-matter of the present invention is further constituted by a strip-like assembly comprising a plurality of single-dose receptacles of the above-indicated type, wherein the above-mentioned receptacles are arranged side by side with the respective longitudinal axes parallel and are interconnected at the upper end and centrally by respective series of tabs provided with at least one predetermined line of fracture.

Advantages and features of the present invention will become clear from the detailed description below which is provided purely by way of non-limiting example with reference to the appended drawings, in which:
Figure 1 is an elevational view of a receptacle of the invention, whose upper portion is shown in cross-section,
Figure 2 is a cross-section through a detail of the receptacle of Figure 1,
Figures 3 and 4 are elevational views of the receptacle of Figure 1 in respective operating configurations and
Figure 5 is a perspective view of a strip-like assembly of receptacles of the invention.

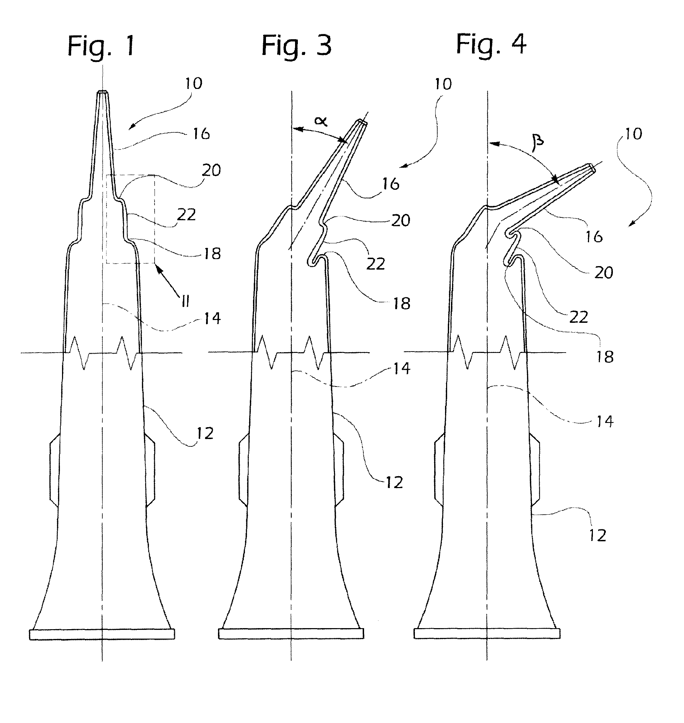

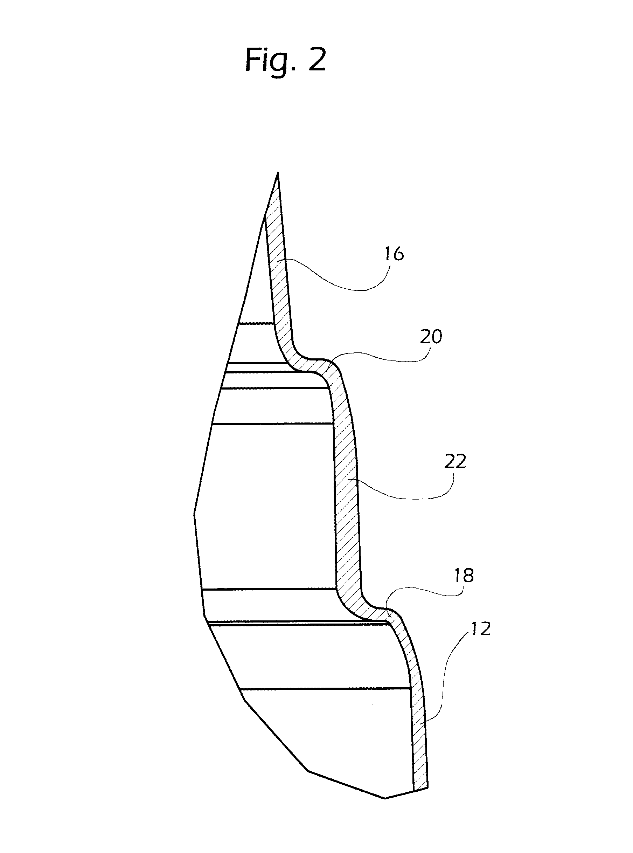

A single-dose receptacle 10 for containing pharmaceutical and cosmetic products and the like comprises (Figure 1) an oblong hollow body 12 having a longitudinal axis 14, the body being of substantially frustoconical shape and having at one end a dispensing mouth 16. The receptacle 10 is typically constructed from plastics material.

The body 12 has (Figure 2) in the region of its end directed towards the mouth 16, a first and a second annular wall portion 18, 20 having reduced thickness and having a cross-section in the form of an inverted L, with a first arm substantially orthogonal to the axis 14 and a second arm which is slightly inclined with respect to the vertical. The second annular portion 20 has a diameter less than that of the first annular portion 18 and is connected thereto by a portion of substantially cylindrical wall 22. The reductions in thickness brought about in the two annular portions 18, 20 act as means allowing the mouth 16 to be inclined with respect to the longitudinal axis 14 at two discrete angles α, β having a predetermined magnitude in the order of approximately 30° and approximately 60°, respectively.

In order to bring about the first inclination, it is simply necessary (Figure 3) for the user to bend the mouth 16 and the cylindrical wall portion 22 together with respect to the body 12. In order to bring about the second inclination, it is instead necessary (Figure 4) for the user to bend the mouth 16 further with respect to the cylindrical wall portion 22.

These inclinations can be effected in any of the planes belonging to the sheaf which extends through the longitudinal axis 14, that is to say, through a complete angle of rotation of 360° about that axis.

The facility to select the plane in which the inclination of the mouth 16 is effected, as well as the magnitude of the inclination, allows the receptacle 10 to be adapted to the specific requirements of the application, also allowing locations which are accessible with difficulty to be reached effectively.

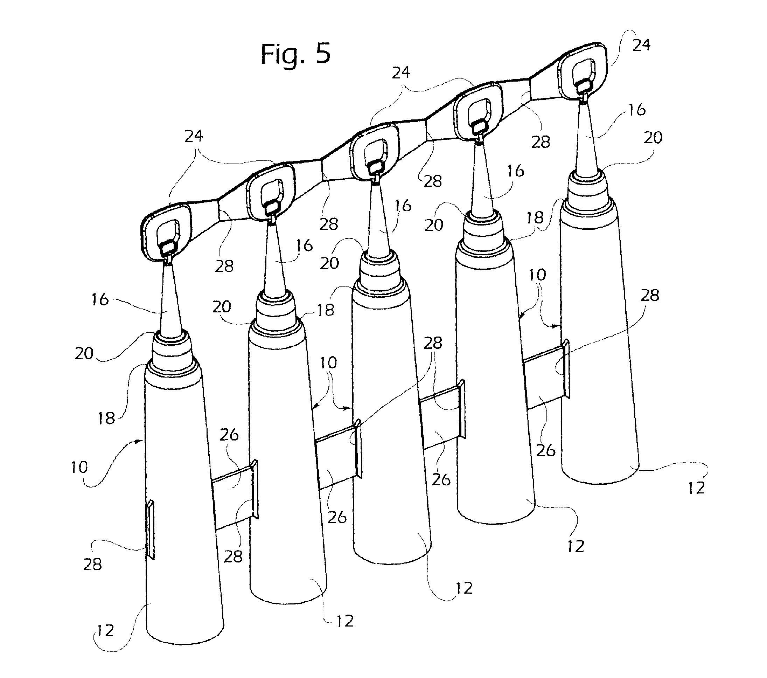

The single-dose receptacles are generally produced and marketed in strip-like assemblies of five. This method of packaging is readily applicable to the receptacles 10 of the present invention, as illustrated in Figure 5.

In the strip-like assembly illustrated, the receptacles 10 are arranged side by side with the respective longitudinal axes parallel and are interconnected at the upper end and centrally by series of tabs 24, 26 provided with predetermined fracture lines 28, respectively, which allow a receptacle 10 which is to be used to be detached from the strip-like assembly from time to time. The fracture lines 28 of the central tabs 26 are produced, insofar as possible, flush with the wall of the bodies 12 of the receptacles 10, so as to prevent disadvantageous protuberances which would impede the handling thereof. The receptacles 10 of Figure 5 are in an intermediate phase of the relevant production process, having the lower end that is opposite the mouth 16 still open, so as to allow subsequent filling of the body 12 with the product in question.

Naturally, the principle of the invention remaining unchanged, the forms of embodiment and details of construction may be varied widely with respect to those described, which have been given purely by way of example, without thereby departing from its scope. In particular, the means which allow the mouth to be inclined with respect to the longitudinal axis of the body can be constructed in such a manner as to allow such inclination to be at an angle of a freely selectable magnitude within a continuous range, or corresponding to a single angular value or a number greater than two.

## Claims

1. A single-dose receptacle (10) for containing pharmaceutical and cosmetic products, and the like, comprising a hollow body (12) having a longitudinal axis (14) and having, at one end, a dispensing mouth (16), said receptacle (10) being **characterized in that** it comprises means which can allow the mouth (16) to be inclined with respect to said longitudinal axis (14).

2. A receptacle (10) according to claim 1, **characterized in that** said means allow the mouth (16) to be inclined with respect to the longitudinal axis (14) at at least one angle (α, β) having a predetermined magnitude.

3. A receptacle (10) according to claim 2, **characterized in that** said means allow the mouth (16) to be inclined with respect to the longitudinal axis (14) at a plurality of discrete angles (α, β) having a predetermined magnitude.

4. A receptacle (10) according to claim 3, **characterized in that** said angles (α, β) are two and have a magnitude of approximately 30° and approximately 60°, respectively.

5. A receptacle (10) according to any one of the preceding claims, **characterized in that** said means allow the mouth (16) to be inclined at least one angle having a predetermined magnitude with respect to the longitudinal axis (14) in any one of the planes belonging to the sheaf extending through said longitudinal axis (14).

6. A receptacle (10) according to any one of the preceding claims, **characterized in that** said body (12) has, in the region of its end directed towards the mouth (16), a first annular wall portion (18) of reduced thickness and having a cross-section which is substantially in the form of an inverted L.

7. A receptacle (10) according to claim 6, **characterized in that** the body (12) has, in the region of its end directed towards the mouth (16), a second annular wall portion (20) of reduced thickness and having a cross-section which is substantially in the form of an inverted L, said second annular portion (20) having a diameter which is less than that of the first annular portion (18) and being connected to the first annular portion (18) by a substantially cylindrical wall portion (22).

8. A receptacle (10) according to any one of the preceding claims, **characterized in that** the body (12) is oblong and of substantially frustoconical shape.

9. A receptacle (10) according to claim 1 or 2, **characterized in that** said means allow the mouth (16) to be inclined with respect to the longitudinal axis (14) at an angle which is of the desired magnitude and which can be selected within a continuous range.

10. A strip-like assembly comprising a plurality of single-dose receptacles (10) according to any one of the preceding claims, wherein the receptacles (10) are arranged side by side with the respective longitudinal axes (14) parallel and are interconnected at the upper end and centrally by respective series of tabs (24, 26) which are provided with at least one predetermined fracture line (28).
